(19) Europäisches Patentamt
European Patent Office
Office européen des brevets

(11) Veröffentlichungsnummer: **0 451 651 A1**

(12) # EUROPÄISCHE PATENTANMELDUNG

(21) Anmeldenummer: **91105111.8**

(51) Int. Cl.5: **C07D 233/50**, A01N 43/50

(22) Anmeldetag: **30.03.91**

(30) Priorität: **12.04.90 DE 4011870**

(43) Veröffentlichungstag der Anmeldung:
**16.10.91 Patentblatt 91/42**

(84) Benannte Vertragsstaaten:
**BE CH DE FR GB IT LI NL**

(71) Anmelder: **BAYER AG**

**W-5090 Leverkusen 1 Bayerwerk(DE)**

(72) Erfinder: **Draber, Wilfried, Dr.**
**In den Birken 81**
**W-5600 Wuppertal(DE)**
Erfinder: **Babczinsky, Peter, Dr.**
**In der Lohrenbeck 11**
**W-5600 Wuppertal(DE)**
Erfinder: **Santel, Hans-Joachim, Dr.**
**Grünstrasse 9a**
**W-5090 Leverkusen(DE)**
Erfinder: **Lürssen, Klaus, Dr.**
**August-Kierspel-Strasse 145**
**W-5060 Bergisch Gladbach 2(DE)**
Erfinder: **Schmidt, Robert R., Dr.**
**Im Waldwinkel 110**
**W-5060 Bergisch Gladbach 2(DE)**

(54) **Imidazolidinderivate.**

(57) Neue Imidazolidinderivate der allgemeinen Formel (I)

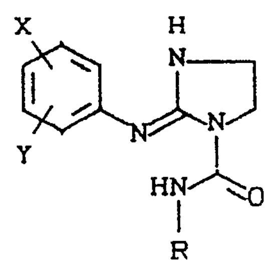

(I)

in welcher

R für jeweils gegebenenfalls substituiertes Alkyl, Cycloalkyl oder Aryl steht und

X und Y unabhängig voneinander für Wasserstoff, Halogen, Cyano, Nitro, Carboxy oder für jeweils gegebenenfalls substituiertes Alkyl, Alkoxy, Alkoxycarbonyl, Alkylthio, Alkylsulfinyl oder Alkylsulfonyl stehen,

ein Verfahren zu ihrer Herstellung und ihre Verwendung als Herbizide.

EP 0 451 651 A1

Die Erfindung betrifft neue Imidazolidinderivate, ein Verfahren zu ihrer Herstellung und ihre Verwendung als Herbizide.

Es wurden neue Imidazolidinderivate der allgemeinen Formel (I)

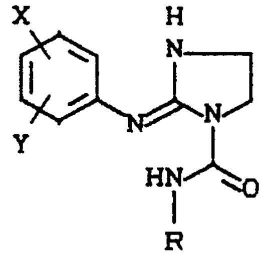

(I)

in welcher

R für jeweils gegebenenfalls substituiertes Alkyl, Cycloalkyl oder Aryl steht und

X und Y unabhängig voneinander für Wasserstoff, Halogen, Cyano, Nitro, Carboxy oder für jeweils gegebenenfalls substituiertes Alkyl, Alkoxy, Alkoxycarbonyl, Alkylthio, Alkylsulfinyl oder Alkylsulfonyl stehen,

gefunden.

Weiter wurde gefunden, daß man die neuen Imidazolidinderivate der Formel (I) erhält, wenn man Imidazolidine der allgemeinen Formel (II)

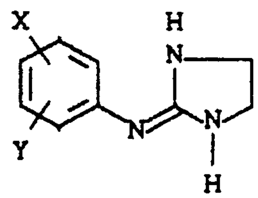

(II)

in welcher

X und Y die oben angegebene Bedeutung haben,

und/oder Tautomere zu den Imidazolidinen der Formel (II) mit Isocyanaten der allgemeinen Formel (III)

R-N=C=O (III)

in welcher

R die oben angegebene Bedeutung hat,

gegebenenfalls in Gegenwart eines Verdünnungsmittels umsetzt.

Schließlich wurde gefunden, daß die neuen Imidazolidinderivate der Formel (I) interessante herbizide Eigenschaften aufweisen.

Die erfindungsgemäßen Imidazolidinderivate sind durch die Formel (I) allgemein definiert. Vorzugsweise stehen in Formel (I)

R für gegebenenfalls durch Halogen substituiertes, geradkettiges oder verzweigtes Alkyl mit 1 bis 6, vorzugsweise 1 bis 4 Kohlenstoffatomen, für Cycloalkyl mit 3 bis 8, vorzugsweise 3 bis 6 Kohlenstoffatomen, welches gegebenenfalls durch geradkettiges oder verzweigtes Alkyl mit 1 bis 4 Kohlenstoffatomen, insbesondere Methyl oder Ethyl, substituiert ist, oder für Phenyl, welches gegebenenfalls durch Cyano, Nitro, Halogen oder für jeweils gegebenenfalls durch Halogen substituiertes Alkyl, Alkoxy oder Alkylthio mit jeweils 1 bis 4 Kohlenstoffatomen substituiert ist, und

X und Y unabhängig voneinander für Wasserstoff, Halogen, Cyano, Nitro, Carboxy oder für jeweils gegebenenfalls durch Halogen substituiertes Alkyl, Alkoxy, Alkoxycarbonyl, Alkylthio, Alkylsulfinyl oder Alkylsulfonyl mit jeweils bis zu 4 Kohlenstoffatomen.

Besonders bevorzugt sind die Verbindungen der Formel (I), in welcher

R für Methyl, Ethyl, Propyl, Isopropyl, Butyl, Isobutyl, sec-Butyl, tert-Butyl, Chlor-tert-butyl, Fluor-tert-butyl, Cyclopentyl, Methyl-cyclopentyl, Ethyl-cyclopentyl, Cyclohexyl, Methyl-cyclohexyl, Ethyl-cyclohexyl oder für Phenyl steht, welches gegebenenfalls einfach, zweifach oder dreifach gleich oder verschieden durch Cyano, Nitro, Fluor, Chlor, Brom, Methyl, Ethyl, Difluormethyl, Chlordifluormethyl, Trifluormethyl, Methoxy, Ethoxy, Difluormethoxy, Chlordifluormethoxy, Trifluormethoxy, Methylthio, Ethylthio, Difluormethylthio, Chlordifluormethylthio oder Trifluormethylthio substituiert ist, und

X und Y unabhängig voneinander für Wasserstoff, Fluor, Chlor, Brom, Cyano, Nitro, Carboxy, Methyl, Ethyl, Difluormethyl, Chlordifluormethyl, Trifluormethyl, Methoxy, Ethoxy, Propoxy, Isopropoxy, Difluormethoxy, Chlordifluormethoxy, Trifluormethoxy, Methoxycarbonyl, Ethoxycarbonyl, Propoxycarbonyl, Isopropoxycarbonyl, Methylthio, Ethylthio, Difluormethylthio, Trifluormethylthio, Chlordifluormethylthio, Methylsulfinyl, Ethylsulfinyl, Trifluormethylsulfinyl, Chlordifluormethylsulfinyl, Trifluormethylsulfonyl oder Chlordifluormethylsulfonyl stehen, wobei X insbesondere in 3-Phenyl-Position steht.

R steht insbesondere für einfach oder zweifach substituiertes Phenyl, wobei als Substituenten Nitro, Fluor, Chlor, Methyl, Ethyl, n- oder iso-Propyl oder Trifluormethyl, Trifluormethoxy oder Trifluormethylthio ausgewählt sind.

X steht insbesondere für Wasserstoff, Trifluormethyl, Methyl, Methoxy, Ethoxy, Propoxy oder Isopropoxy, wobei die Substituenten vorzugsweise in 3-Phenyl-Position stehen.

Y steht insbesondere für Wasserstoff.

Beispiele für die Verbindungen der Formel (I) sind in der nachstehenden Tabelle 1 aufgeführt.

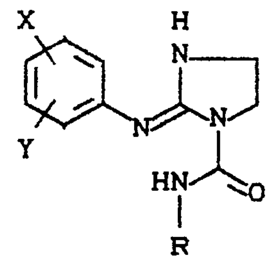

(I)

Tabelle 1: Beispiele für die Verbindungen der Formel (I)

| R | (Position-)X | (Position-)Y |
|---|---|---|
| $-CH(CH_3)C_2H_5$ | (3-)Cl | H |
| 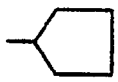 | (2-)F | (4-)Cl |
| $C_2H_5$ | (3-)Cl | (5-)Cl |
| 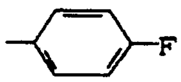 | (3-)$CF_3$ | H |
| 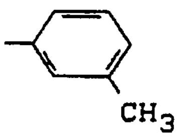 | (4-)F | H |
| 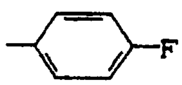 | (3-)$OCF_3$ | H |
| 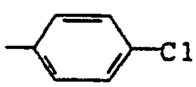 | (3-)$CF_3$ | H |
| 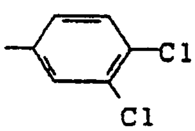 | (3-)$O-CH(CH_3)_2$ | H |
| 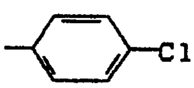 | (3-)$OCH(CH_3)_2$ | H |
| 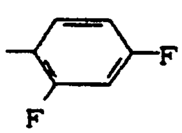 | (3-)$OCH_3$ | H |
| 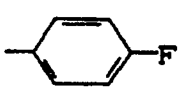 | (4-)$CO_2CH_3$ | H |

Tabelle 1 - Fortsetzung

| R | (Position-)X | (Position-)Y |
| --- | --- | --- |
| 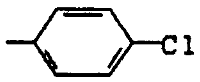 | (3-)$CO_2CH(CH_3)_2$ | H |
| 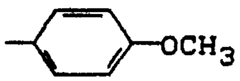 | (3-)F | (4-)Cl |

Verwendet man für das erfindungsgemäße Verfahren zur Herstellung der Verbindungen der Formel (I) beispielsweise 2-(3-Cyano-phenyl-imino)-imidazolidin und 2-Fluorphenylisocyanat als Ausgangsstoffe, so kann der Reaktionsablauf durch das folgende Formelschema wiedergegeben werden:

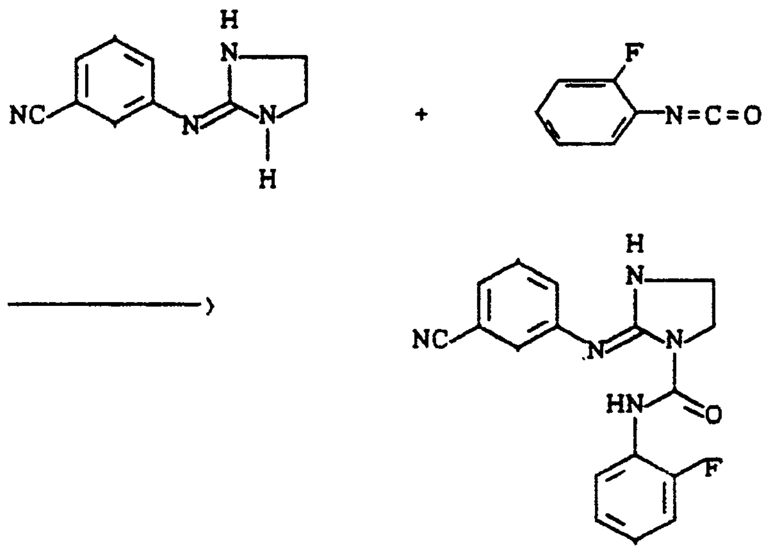

Die beim erfindungsgemäßen Verfahren zur Herstellung von Verbindungen der Formel (I) als Ausgangsstoffe zu verwendenden Imidazolidine sind durch die Formel (II) allgemein definiert.

In Formel (II) haben X und Y vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für X und Y angegeben wurden.

Die Ausgangsstoffe der Formel (II) - bzw. deren Tautomere - sind bekannt und/oder können nach an sich bekannten Verfahren hergestellt werden (vgl. J. Org. Chem. 24 (1959), 884; J. Het. Chem. 11 (1974), 257-262; DE-P 1303930; GB-P 1382752).

Man erhält die Imidazolidine der Formel (II) beispielsweise, wenn man 2-Chlor-imidazolin der Formel (IV)

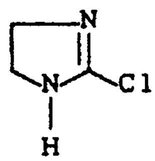 (IV)

oder Salze hiervon, mit Arylaminen der allgemeinen Formel (V)

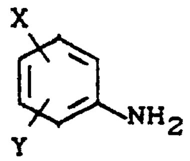

(V)

in welcher

X und Y die oben angegebene Bedeutung haben,

in Gegenwart eines Säurebindemittels, wie z.B. Natriumhydroxid und in Gegenwart eines Verdünnungsmittels, wie z.B. Methylenchlorid, bei Temperaturen zwischen 0 °C und 100 °C umsetzt.

2-Chlor-imidazolin und dessen Salze sind bekannt (vgl. J. Het. Chem. 11 (1974), 257-262).

Die Arylamine der Formel (V) sind ebenfalls bekannte Synthesechemikalien.

Die beim erfindungsgemäßen Verfahren zur Herstellung von Verbindungen der Formel (I) weiter als Ausgangsstoffe zu verwendenden Isocyanate sind durch die Formel (III) allgemein definiert.

In Formel (III) hat R vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für R angegeben wurde.

Die Ausgangsstoffe der Formel (III) sind bekannte organische Synthesechemikalien bzw. können nach bekannten Verfahren hergestellt werden.

Das erfindungsgemäße Verfahren zur Herstellung der neuen Imidazolidinderivate der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Die Reaktionstemperaturen können bei dem erfindungsgemäßen Verfahren in einem größeren Bereich variiert werden. Im allgemeinen arbeitet man bei Temperaturen zwischen -20 °C und +150 °C, vorzugsweise bei Temperaturen zwischen 0 °C und 100 °C.

Das erfindungsgemäße Verfahren wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel durchgeführt, und das Reaktionsgemisch wird mehrere Stunden bei der jeweils erforderlichen Temperatur gerührt. Die Aufarbeitung erfolgt bei dem erfindungsgemäßen Verfahren jeweils nach üblichen Methoden (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich beispielsweise zur selektiven Bekämpfung von monokotylen und dikotylen Unkräutern in dikotylen Kulturen, wie Soja oder Sonnenblumen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, infrage; ferner auch Chloressigsäure-N-(methoxymethyl)-2,6-diethylanilid (ALACHLOR); Ethyl-2-{[(4-chlor-6-methoxy-2-pyrimidinyl)-aminocarbonyl]-aminosulfonyl}-benzoat (CHLORIMURON); exo-1-Methyl-4-(1-methylethyl)-2-(2-methylphenyl-methoxy)-7-oxabicyclo-(2,2,1)-heptan (CINMETHYLIN); 2-[(2-Chlorphenyl)-methyl]-4,4-dimethylisoxazolidin-3-on (DIMETHAZONE); 4-Amino-6-t-butyl-3-ethylthio-1,2,4-triazin-5(4H)-on (ETHIOZIN); 2-[5-Methyl-5-(1-methylethyl)-4-oxo-2-imidazolin-2-yl]-3-chinolincarbonsäure (IMAZAQUIN); 2-[4,5-Dihydro-4-methyl-4-isopropyl-5-oxo-(1H)-imidazol-2-yl]-5-ethyl-pyridin-3-carbonsäure (IMAZETHAPYR); N-Methyl-2-(1,3-benzthiazol-2-yloxy)-acetanilid (MEFENACET); 2-Chlor-N-(2,6-dimethylphenyl)-N-[(1H)-pyrazol-1-yl-methyl]-acetamid (METAZACHLOR); 2-Ethyl-6-methyl-N-(1-methyl-2-methoxyethyl)-chloracetanilid (METOLACHLOR); 3-[[[[(4-Methoxy-6-methyl-1,3,5-triazin-2-yl)-amino]-carbonyl]-amino]-sulfonyl]-thiophen-2-carbonsäure-methylester (THIAMETURON); 2,6-Dinitro-4-trifluormethyl-N,N-dipropylanilin (TRIFLURALIN). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken, Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

## Herstellungsbeispiele:

### Beispiel 1

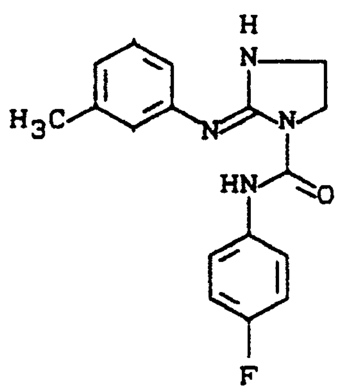

Eine Lösung von 13,7 g (0,1 Mol) 4-Fluor-phenylisocyanat in 25 ml Methylenchlorid wird unter Rühren zu einer mit Eis gekühlten Lösung von 17,5 g (0,1 Mol) 2-(3-Methylphenylimino)-imidazolidin in 250 ml Methylenchlorid tropfenweise gegeben. Nach Entfernen des Eisbads wird das Reaktionsgemisch 3 Stunden bei 10° C bis 20° C gerührt und anschließend filtriert. Das Filtrat wird mit Natriumsulfat getrocknet, eingeengt, der Rückstand mit Diethylether verrührt, das kristalline Produkt durch Absaugen isoliert und aus Essigsäureethylester umkristallisiert.

Man erhält 25,0 g (80% der Theorie) 1-(4-Fluor-phenylamino-carbonyl)-2-(3-methyl-phenylimino)-imidazolidin vom Schmelzpunkt 178° C.

Analog Beispiel 1 und entsprechend der allgemeinen Beschreibung des erfindungsgemäßen Herstellungsverfahrens können beispielsweise auch die in der nachstehenden Tabelle 2 aufgeführten Verbindungen der Formel (I) hergestellt werden.

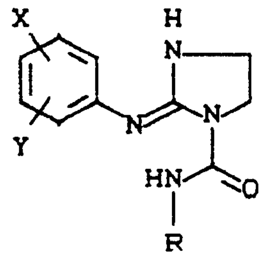

(I)

Tabelle 2: Beispiele für die Verbindungen der Formel (I)

| Bsp.-Nr. | R | (Position-)X | (Position-)Y | Schmelzpunkt (°C) |
|---|---|---|---|---|
| 2 | 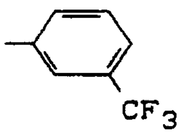 | H | H | 138 |
| 3 | 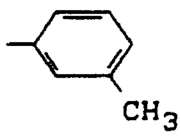 | H | H | 189 |
| 4 | 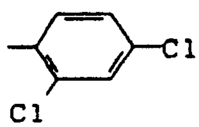 | (3-)$CF_3$ | H | 150 |
| 5 | 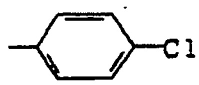 | (3-)$CF_3$ | H | 166 |

Tabelle 2 - Fortsetzung

| Bsp.-Nr. | R | (Position-)X | (Position-)Y | Schmelzpunkt ($^{0}C$) |
|---|---|---|---|---|
| 6 | 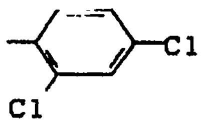 | (3-)$CH_3$ | H | 181 |
| 7 | 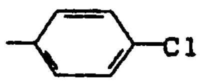 | (3-)$CH_3$ | H | 188 |
| 8 | 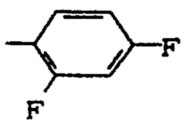 | (3-)$CH_3$ | H | 175 |
| 9 | 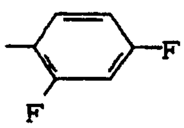 | (3-)$CF_3$ | H | 198 |
| 10 | 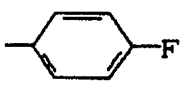 | (3-)$CF_3$ | H | 180 |
| 11 | 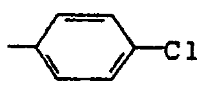 | (3-)$OCH_3$ | H | 163 |
| 12 | 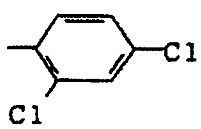 | (3-)$OCH_3$ | H | 160 |
| 13 | 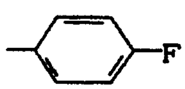 | (3-)$OCH_3$ | H | 156 |

Tabelle 2 - Fortsetzung

| Bsp.-Nr. | R | (Position-)X | (Position-)Y | Schmelzpunkt (°C) |
|---|---|---|---|---|
| 14 | 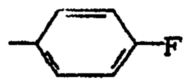 | (3-)$OCH(CH_3)_2$ | H | 157 |
| 15 | 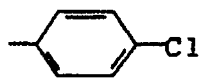 | (3-)$OCH(CH_3)_2$ | H | 165 |
| 16 | 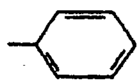 | (3-)$OCH(CH_3)_2$ | H | 148 |
| 17 | 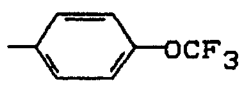 | (3-)$OCH(CH_3)_2$ | H | 177 |
| 18 | 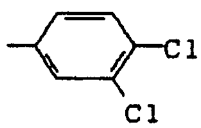 | (3-)$OCH(CH_3)_2$ | H | 122 |
| 19 | 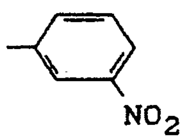 | (3-)$OCH(CH_3)_2$ | H | 123 |
| 20 | 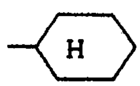 | (3-)$OCH(CH_3)_2$ | H | 164 |
| 21 | 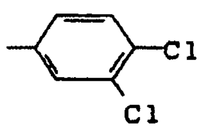 | (3-)$OCH_3$ | H | 167 |
| 22 | 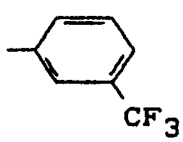 | (3-)$OCH(CH_3)_2$ | H | 115 |
| 23 | 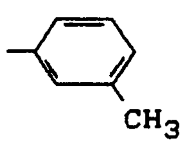 | (3-)$OCH(CH_3)_2$ | H | 111 |

Tabelle 2 - Fortsetzung

| Bsp.-Nr. | R | (Position-)X | (Position-)Y | Schmelzpunkt (°C) |
|---|---|---|---|---|
| 24 | 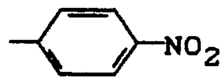 | (3-)$OCH(CH_3)_2$ | H | 175 |
| 25 | 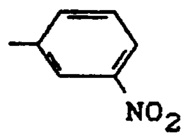 | (3-)$CH_3$ | H | 165 |
| 26 | 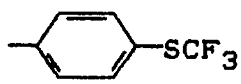 | (3-)$OCH(CH_3)_2$ | H | 164 |
| 27 | 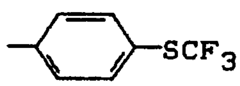 | (3-)$OCH_3$ | H | 135 |
| 28 | 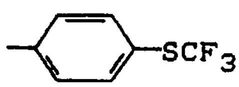 | (3-)$CF_3$ | H | 152 |

Ausgangsstoffe der Formel (II):

Beispiel (II-1)

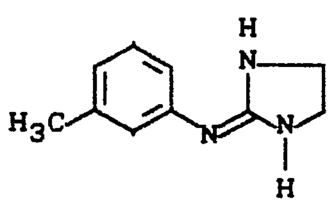

Eine Lösung von 60 g (1,5 Mol) Natriumhydroxid in 300 ml Wasser wird bei 20 °C tropfenweise zu einer Mischung aus 94,2 g (0,5 Mol) 2-Chlor-imidazolinsulfat, 53.5 g 3-Methyl-anilin und 1 Liter Methylenchlorid gegeben und das Reaktionsgemisch wird 20 Stunden bei 20 °C gerührt. Anschließend wird die organische Phase abgetrennt, mit Natriumsulfat getrocknet und filtriert. Das Filtrat wird eingeengt, der Rückstand durch Verreiben mit Diethylether zur Kristallisation gebracht und das kristalline Produkt durch Absaugen isoliert.

Man erhält 34 g (39% der Theorie) 2-(3-Methyl-phenylimino)-imidazolidin vom Schmelzpunkt 115 °C.

Analog Beispiel (II-1) können beispielsweise auch die in der nachstehenden Tabelle 3 aufgeführten Verbindungen der Formel (II) hergestellt werden.

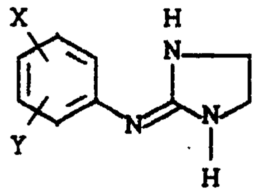

(II)

**Tabelle 3:** Beispiele für die Ausgangsstoffe der Formel (II)

| Bsp.-Nr. | X | Y | Schmelzpunkt (°C) |
|---|---|---|---|
| II-2 | (3-)$CF_3$ | H | 93 |
| II-3 | (3-)$OCH_3$ | H | 97 |
| II-4 | (3-)$OCF_3$ | H | 102 |

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigen z.B. die Verbindungen gemäß den Herstellungsbeispielen 8, 9, 10 und 13 bei guter Verträglichkeit gegenüber Kulturpflanzen, wie z.B. Soja und Sonnenblumen, starke Wirkung gegen Unkräuter.

**Patentansprüche**

**1.** Imidazolidinderivate der allgemeinen Formel (I)

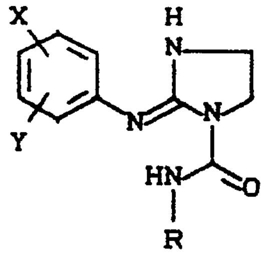

(I)

in welcher

R für jeweils gegebenenfalls substituiertes Alkyl, Cycloalkyl oder Aryl steht und

X und Y unabhängig voneinander für Wasserstoff, Halogen, Cyano, Nitro, Carboxy oder für jeweils gegebenenfalls substituiertes Alkyl, Alkoxy, Alkoxycarbonyl, Alkylthio, Alkylsulfinyl oder Alkylsulfonyl stehen.

2. Imidazolidinderivate der Formel (I) gemäß Anspruch 1,

in welcher

R für gegebenenfalls durch Halogen substituiertes, geradkettiges oder verzweigtes Alkyl mit 1 bis 6 Kohlenstoffatomen, für Cycloalkyl mit 3 bis 8 Kohlenstoffatomen steht, welches gegebenenfalls durch geradkettiges oder verzweigtes Alkyl mit 1 bis 4 Kohlenstoffatomen substituiert ist, oder für Phenyl steht, welches gegebenenfalls durch Cyano, Nitro, Halogen oder für jeweils gegebenenfalls durch Halogen substituiertes Alkyl, Alkoxy oder Alkylthio mit jeweils 1 bis 4 Kohlenstoffatomen substituiert ist, und

X und Y unabhängig voneinander für Wasserstoff, Halogen, Cyano, Nitro, Carboxy oder für jeweils gegebenenfalls durch Halcgen substituiertes Alkyl, Alkoxy, Alkoxycarbonyl, Alkylthio, Alkylsulfinyl oder Alkylsulfonyl mit jeweils bis zu 4 Kohlenstoffatomen steht.

3. Imidazolidinderivate der Formel (I) gemäß Anspruch 1,

in welcher

R für Methyl, Ethyl, Propyl, Isopropyl, Butyl, Isobutyl, sec-Butyl, tert-Butyl, Chlor-tert-butyl, Fluor-tert-butyl, Cyclopentyl, Methyl-cyclopentyl, Ethyl-cyclopentyl, Cyclohexyl, Methylcyclohexyl, Ethyl-cyclohexyl oder für Phenyl steht, welches gegebenenfalls einfach, zweifach oder dreifach gleich oder verschieden durch Cyano, Nitro, Fluor, Chlor, Brom, Methyl, Ethyl, Difluormethyl, Chlordifluormethyl, Trifluormethyl, Methoxy, Ethoxy, Difluormethoxy, Chlordifluormethoxy, Trifluormethoxy, Methylthio, Ethylthio, Difluormethylthio, Chlordifluormethylthio oder Trifluormethylthio substituiert ist, und

X und Y unabhängig voneinander für Wasserstoff, Fluor, Chlor, Brom, Cyano, Nitro, Carboxy, Methyl, Ethyl, Difluormethyl, Chlordifluormethyl, Trifluormethyl, Methoxy, Ethoxy, Propoxy, Isopropoxy, Difluormethoxy, Chlordifluormethoxy, Trifluormethoxy, Methoxycarbonyl, Ethoxycarbonyl, Propoxycarbonyl, Isopropoxycarbonyl, Methylthio, Ethylthio, Difluormethylthio, Trifluormethylthio, Chlordifluormethylthio, Methylsulfinyl, Ethylsulfinyl, Trifluormethylsulfinyl, Chlordifluormethylsulfinyl, Trifluormethylsulfonyl oder Chlordifluormethylsulfonyl stehen.

4. Imidazolidinderivate der Formel (I) gemäß Anspruch 1, in welcher R für einfach oder zweifach substituiertes Phenyl steht, wobei als Substituenten Fluor, Chlor, Methyl, Ethyl, n- oder iso-Propyl oder Trifluormethyl ausgewählt sind.

5. Verfahren zur Herstellung von Imidazolidinderivaten der allgemeinen Formel (I)

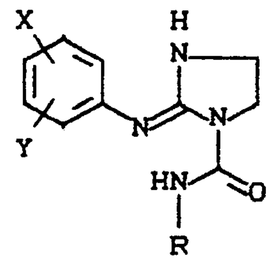

(I)

in welcher

R für jeweils gegebenenfalls substituiertes Alkyl, Cycloalkyl oder Aryl steht und

X und Y unabhängig voneinander für Wasserstoff, Halogen, Cyano, Nitro, Carboxy oder für jeweils gegebenenfalls substituiertes Alkyl, Alkoxy, Alkoxycarbonyl, Alkylthio, Alkylsulfinyl oder Alkylsulfonyl stehen,

dadurch gekennzeichnet, daß man Imidazolidine der allgemeinen Formel (II)

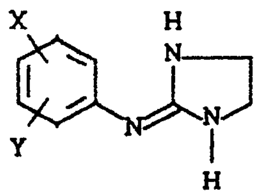

(II)

in welcher

X und Y die oben angegebene Bedeutung haben, und/oder Tautomere zu den Imidazolidinen der Formel (II) mit Isocyanaten der allgemeinen Formel (III)

R-N=C=O (III)

in welcher

R die oben angegebene Bedeutung hat,

gegebenenfalls in Gegenwart eines Verdünnungsmittels umsetzt.

6. Herbizide Mittel, gekennzeichnet durch einen Gehalt an mindstens einem Imidazolidinderivat der Formel (I) gemäß Anspruch 1 oder 5.

7. Verfahren zur Bekämpfung von Unkräutern, dadurch gekennzeichnet, daß man Imidazolidinderivate der Formel (I) gemäß Anspruch 1 oder 5 auf die Unkräuter und/oder ihren Lebensraum einwirken läßt.

8. Verwendung von Imidazolidinderivaten der Formel (I) gemäß Anspruch 1 oder 5 zur Bekämpfung von Unkräutern.

9. Verfahren zur Herstellung von herbiziden Mitteln, dadurch gekennzeichnet, daß man Imidazolidinderivate der Formel (I) gemäß Anspruch 1 oder 5 mit Streckmitteln und/oder oberflächenaktiven Substanzen vermischt.

Europäisches Patentamt

# EUROPÄISCHER RECHERCHENBERICHT

Nummer der Anmeldung

EP 91105111.8

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.⁵) |
| A | US - A - 4 262 005 (MC CARTHY et al.) * Zusammenfassung * -- | 1,5 | C 07 D 233/50<br>A 01 N 43/50 |
| A | CHEMICAL ABSTRACTS, Band 96, Nr. 17, 26. April 1982, Columbus, Ohio, USA FBC LTD "Pesticide composition and method for controlling noxions inseet pests on domestic animals" Seite 321, Spalte 1, Zusammenfassung-Nr. 138 024z & Braz. Pedido PI BR 81 01 938 -- | 1,5 | |
| A | GB - A - 2 010 816 (ELI LILLY) * Zusammenfassung * ---- | 1,5 | |

RECHERCHIERTE SACHGEBIETE (Int. Cl.⁵)

C 07 D 233/00

Der vorliegende Recherchenbericht wurde für alle Patentansprüche erstellt.

| Recherchenort | Abschlußdatum der Recherche | Prüfer |
|---|---|---|
| WIEN | 16-05-1991 | BRUS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 62